# EUROPEAN PATENT APPLICATION

(11) **EP 4 098 327 A1**
(43) Date of publication of application: **07.12.2022**
(21) Application number: 21748143.1
(22) Date of filing: 29.01.2021
(51) Int. Cl.: A61Q 17/04, A61K 8/06, A61K 8/29, A61K 8/31, A61K 8/891

(54) **WATER-IN-OIL EMULSION COSMETIC**

(30) Priority: 31.01.2020 JP 2020015174
(71) Applicant: Kao Corporation, Chuo-ku Tokyo 103-8210 (JP)
(72) Inventor: CHEN, Shi, Tokyo 131-8501 (JP); ISHITA, Mio, Tokyo 131-8501 (JP)
(74) Representative: Hoffmann Eitle
(86) International application number: PCT/JP2021/003137
(87) International publication number: WO 2021/153704

(57) **Abstract**

A water-in-oil emulsion cosmetic, in which an ultraviolet scattering agent is uniformly dispersed in a base, an excellent ultraviolet protective effect is stably imparted, and whitishness is suppressed.

The present invention relates to a water-in-oil emulsion cosmetic comprising the following components (a), (b), (c) and (d) :
(a) titanium dioxide surface-treated with a fatty acid and/or an alkylalkoxysilane;
(b) an amphiphilic substance which is solid at normal temperature;
(c) a linear silicone oil; and
(d) a volatile hydrocarbon oil.

## Description

### Field of the Invention

The present invention relates to a water-in-oil emulsion cosmetic useful as a sunscreen cosmetic.

### Background of the Invention

Sunscreen cosmetics contain ultraviolet absorbers and ultraviolet scattering agents such as titanium dioxide and zinc oxide. Of these, measures have been taken to reduce the amount of contained ultraviolet absorbers due to a demand for cosmetics to be gentler on the skin in recent years.

The cosmetics containing ultraviolet scattering agents such as titanium dioxide causes the skin to turn whitish when ultraviolet scattering agents are contained in an amount to exhibit a sufficient sunscreen effect.

Therefore, cosmetics have been developed to ensure transparency while containing a sufficient amount of ultraviolet scattering agents such as titanium dioxide. For example, there are reports of a sunscreen cosmetic containing titanium dioxide treated with trimethylsiloxysilicate, cross-linked methylpolysiloxane, an ester oil, squalane, and water (Patent Literature 1); a water-in-oil emulsion sunscreen cosmetic containing hydrophobically-treated zinc oxide and hydrophobically-treated titanium dioxide, a lipophilic nonionic surfactant, an oil such as di(phytosteryl/2-octyldodecyl) N-lauroyl-L-glutamate, a volatile silicone oil and/or hydrocarbon oil and water (Patent Literature 2); a water-in-oil sunscreen cosmetic containing polyether modified silicone, titanium dioxide and zinc oxide, a volatile oil, organosiloxane elastomer and cetyl dimethicone, but no ultraviolet absorbers (Patent Literature 3); and a water-in-oil emulsion cosmetic containing a volatile oil, a silicone surfactant, a hydrophobic powder, a silicone film-forming agent, and a hydrophobically-modified polyetherurethane (Patent Literature 4).

(Patent Literature 1) JP-A-2014-91736
(Patent Literature 2) JP-A-2013-43875
(Patent Literature 3) JP-A-2008-208044
(Patent Literature 4) JP-A-2017-178887

### Summary of the Invention

The present invention relates to a water-in-oil emulsion cosmetic comprising the following components (a), (b), (c) and (d) :
(a) titanium dioxide surface-treated with a fatty acid and/or an alkylalkoxysilane;
(b) an amphiphilic substance which is solid at normal temperature;
(c) a chain silicone oil; and
(d) a volatile hydrocarbon oil.

### Brief Description of Drawings

Figure 1 is a micrograph showing the dispersion of the component (a) when the component (b) of the present invention is contained.
Figure 2 is a micrograph showing the dispersion of the component (a) when an amphiphilic substance in a liquid state at normal temperature is contained instead of the component (b) of the present invention.
Figure 3 is a micrograph showing the dispersion of the component (a) when the component (b) of the present invention is not contained.

### Detailed Description of the Invention

In the cosmetics of the above patent literatures, special bases were used such as cross-linked methylpolysiloxane, di(phytosteryl/2-octyldodecyl) N-lauroyl-L-glutamate, organosiloxane elastomer, and hydrophobically-modified polyetherurethane, then the ultraviolet scattering agents such as titanium dioxide were not always well dispersed in the base, a high ultraviolet protective effect could not be stably imparted, and whitishness could not be sufficiently suppressed.

Therefore, the present invention relates to a water-in-oil emulsion cosmetic, in which an ultraviolet scattering agent is uniformly dispersed in a base, an excellent ultraviolet protective effect is stably imparted, and whitishness is suppressed.

As a result of various studies, the present inventors found that by containing a particular hydrophobically-treated titanium dioxide as an ultraviolet scattering agent, combined with an amphiphilic substance which is solid at normal temperature, a chain silicone oil, and a volatile hydrocarbon oil, and uniformly emulsifying and dispersing them, a water-in-oil emulsion cosmetic can be obtained, in which the hydrophobically-treated titanium dioxide is uniformly dispersed in the base, by which an excellent ultraviolet protective effect is stably imparted, whitishness is suppressed, and good spreadability and stability are imparted.

The present invention provides a water-in-oil emulsion cosmetic, in which hydrophobically-treated titanium dioxide is uniformly dispersed in a base, an excellent ultraviolet protective effect is stably imparted, whitishness is suppressed, and good spreadability and stability are imparted.

The water-in-oil emulsion cosmetic of the present invention contains the following components (a), (b), (c) and (d):
(a) titanium dioxide surface-treated with a fatty acid and/or an alkylalkoxysilane;
(b) an amphiphilic substance which is solid at normal temperature;
(c) a linear silicone oil; and
(d) a volatile hydrocarbon oil.

The component(a) is titanium dioxide surface-treated with a fatty acid and/or an alkylalkoxysilane.

Titanium dioxide preferably has an average particle size of 5 nm or more and 100 nm or less, more preferably an average particle size of 5 nm or more and 50 nm or less, and further more preferably an average particle size of 10 nm or more and 30 nm or less from the viewpoint of imparting a high ultraviolet protective effect and suppressing whitishness. Note that in the present invention, the average particle size is obtained by observing the primary particles with a transmission electron microscope and calculating the number average particle size by image analysis thereof.

The titanium dioxide used in the present invention is surface-treated with a fatty acid and/or an alkylalkoxysilane. As the fatty acid used in the treatment, a fatty acid having from 10 to 22 carbon atoms is preferable, a fatty acid having from 12 to 22 carbon atoms is more preferable, and a fatty acid having from 12 to 20 carbon atoms is further more preferable from the viewpoint of the surface-treated titanium dioxide having good dispersibility in the component (b). Specifically, lauric acid, myristic acid, palmitic acid, stearic acid, and isostearic acid are preferably used, and stearic acid and/or isostearic acid are more preferably used. As the alkylalkoxysilane used in the treatment, hexyltrimethoxysilane, octyltrimethoxysilane, octyltriethoxysilane, decyltrimethoxysilane, and octadecyltrimethoxysilane are preferably used, and octyltriethoxysilane is more preferably used from the viewpoint of the surface-treated titanium dioxide having good dispersibility in the component (b)

Titanium dioxide may also be treated with aluminum hydroxide in addition to these surface treatment agents.

The surface treatment of titanium dioxide can be performed, for example, by mixing and stirring titanium dioxide in a fatty acid and/or alkylalkoxysilane for a certain period of time.

A content of the component (a) in the water-in-oil emulsion cosmetic of the present invention is preferably 3 mass% or more, more preferably 5 mass% or more from the viewpoint of the ultraviolet protective effect, and preferably 20 mass% or less, and preferably 15 mass% or less from the viewpoint of suppressing whitishness. Specifically, it is preferably 3 mass% or more and 20 mass% or less, more preferably 5 mass% or more and 20 mass% or less, and further more preferably 5 mass% or more and 15 mass% or less.

The water-in-oil emulsion cosmetic of the present invention may also contain hydrophobically-treated zinc oxide other than (a) titanium dioxide surface-treated with a fatty acid and/or an alkylalkoxysilane.

The zinc oxide in the hydrophobically-treated zinc oxide preferably has an average particle size of 5 nm or more and 100 nm or less, more preferably an average particle size of 5 nm or more and 80 nm or less, and further more preferably an average particle size of 10 nm or more and 50 nm or less from the viewpoint of imparting a high ultraviolet protective effect and suppressing whitishness.

As a means of hydrophobic treatment of hydrophobic zinc oxide, a fatty acid treatment, a silane treatment, a silicone treatment, and the like are preferable, a silane treatment and a silicone treatment are more preferable, and a treatment with an alkylalkoxysilane such as octyltrimethoxysilane and octyltriethoxysilane, and a treatment with a silicone such as dimethicone and methicone are further more preferable.

A content of the hydrophobically-treated zinc oxide in the water-in-oil emulsion cosmetic of the present invention is preferably 5 mass% or more, more preferably 8 mass% or more, and further more preferably 10 mass% or more from the viewpoint of the ultraviolet protective effect, and preferably 30 mass% or less, more preferably 25 mass% or less, and further more preferably 23 mass% or less from the viewpoint of suppressing whitishness. Specifically, it is preferably 5 mass% or more and 30 mass% or less, more preferably 8 mass% or more and 25 mass% or less, and further more preferably 10 mass% or more and 23 mass% or less.

The component(b) is an amphiphilic substance which is solid at normal temperature. Here, normal temperature refers to a range of from 5°C to 35°C. Solid refers to a state in which no fluidity is exhibited and a constant shape is maintained in this temperature range.

Examples of such amphiphilic substance include ceramides, alcohols having from 8 to 22 carbon atoms, glycerol fatty acid esters having from 8 to 22 carbon atoms, alkyl glyceryl ethers having from 8 to 22 carbon atoms, sorbitan fatty acid esters having from 8 to 22 carbon atoms, and polyoxyethylene sorbitan fatty acid esters having from 8 to 22 carbon atoms. In the present invention, one or more selected from the group consisting of these amphiphilic substances can be used.

As the ceramides, one or more selected from the group consisting of natural ceramides and pseudo-ceramides can be used. Specifically, the ceramides described in JP-A-2013-53146 are preferable from the viewpoint of stably dispersing the component (a) to impart a high ultraviolet protective effect, and of imparting a good impression from use.

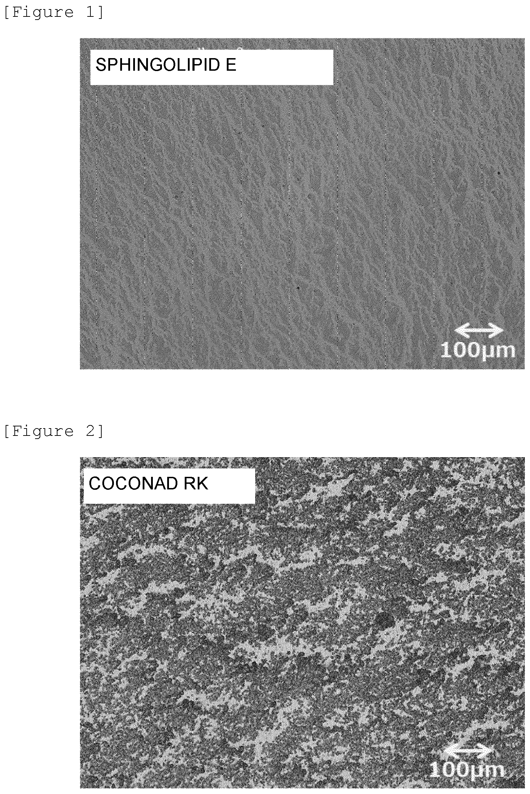

Specific examples of natural ceramides include the ceramides Type 1 to 7 in which sphingosine, dihydrosphingosine, phytosphingosine or sphingadienine is amidated (for example, the porcine and human ceramides described in Figure 2 of J. Lipid Res., 24:759 (1983), and Figure 4 of J. Lipid. Res., 35:2069 (1994)).

Furthermore, the N-alkyl forms thereof (for example, N-methyl form) are also included.

An optically active natural form (D(-) form), an optically active non-natural form (L(+) form), or even a mixture of natural and non-natural forms of these ceramides may be used. The relative stereo configuration of the above described compounds may be a natural stereo configuration, a non-natural stereo configuration other than this, or even a mixture thereof. In particular, the compounds CERAMIDE 1, CERAMIDE 2, CERAMIDE 3, CERAMIDE 5 and CERAMIDE 6 II (all of the above, INCI, 8th Edition), and those of the following formulas are preferable.

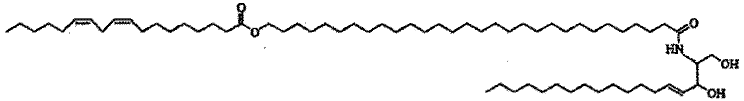

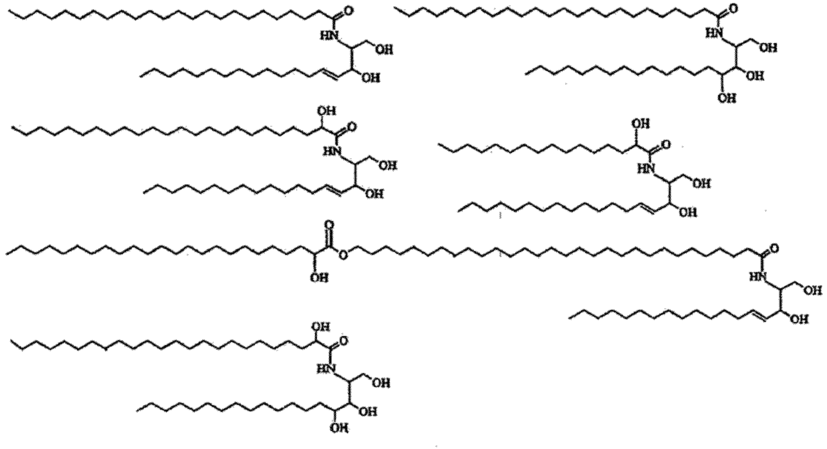

These may be either natural extracts or synthetic products, and commercially available products can be used. Examples of commercially available products of such natural ceramides include Ceramide I, Ceramide III, Ceramide IIIA, Ceramide IIIB, Ceramide IIIC, Ceramide VI (all of the above, COSMO FARM), Ceramide TIC-001 (Takasago International Corporation), CERAMIDE II (Quest International), DS-Ceramide VI, DS-CLA-Phytoceramide, C6-Phytoceramide, DS-ceramide Y3S (DOOSAN), and CERAMIDE 2 (Sederma).

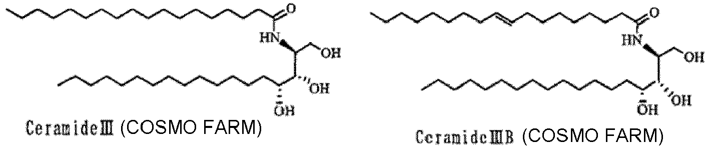

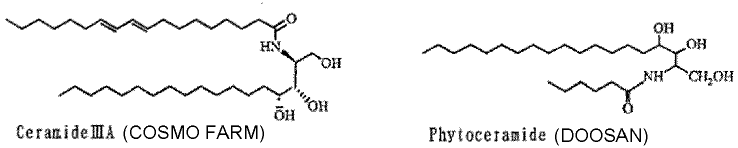

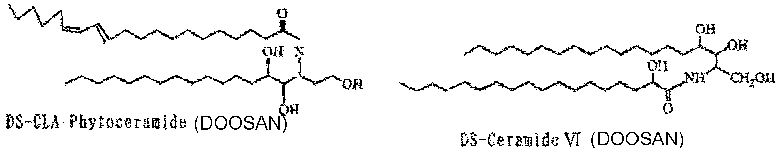

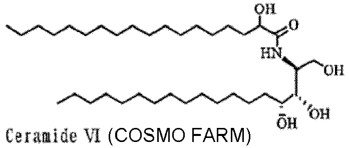

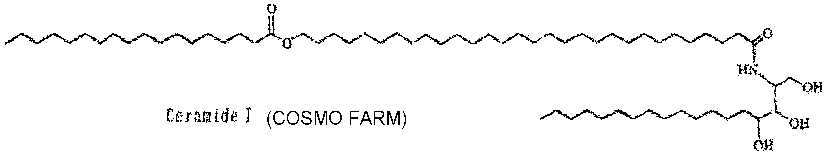

As a pseudo-ceramide, a pseudo-ceramide of the following formula (1) is preferable from the viewpoint of stably dispersing the component (a) to impart a high ultraviolet protective effect, and of imparting a good impression from use: 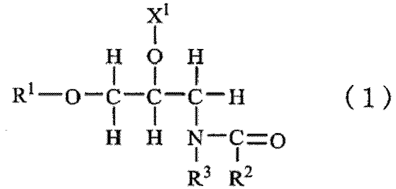 wherein R¹ indicates a linear, branched, or cyclic saturated or unsaturated hydrocarbon group having from 10 to 22 carbon atoms optionally substituted with a hydroxyl group, or a hydrogen atom; X¹ indicates a hydrogen atom, an acetyl group or a glyceryl group; R² indicates a linear, branched, or cyclic saturated or unsaturated hydrocarbon group having from 5 to 22 carbon atoms optionally substituted with a hydroxyl group or an amino group, or the hydrocarbon group to which a linear or branched saturated or unsaturated fatty acid having from 8 to 22 carbon atoms optionally substituted with a hydroxyl group is linked via an ester bond to its ω-terminus; and R³ indicates a hydrogen atom, or an alkyl group having a total of from 1 to 30 carbon atoms optionally substituted with a hydroxyl group, a hydroxyalkoxy group, an alkoxy group or an acetoxy group.

Among these, the pseudo-ceramides of the following formulas are more preferable from the viewpoint of stably dispersing the component (a) to impart a high ultraviolet protective effect, and of imparting a good impression from use.

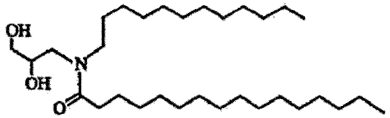

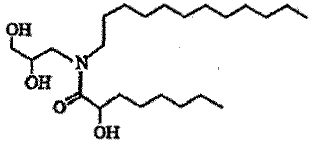

As the formula (1), the pseudo-ceramides of the following formulas are preferable, wherein R¹ is a hexadecyl group, X¹ is a hydrogen atom, R² is a pentadecyl group, and R³ is a hydroxyethyl group; and wherein R¹ is a hexadecyl group, X¹ is a hydrogen atom, R² is a nonyl group, and R³ is a hydroxyethyl group, and further more preferably R¹ is a hexadecyl group, X¹ is a hydrogen atom, R² is a pentadecyl group, and R³ is a hydroxyethyl group in the formula (1) (N-(hexadecyloxyhydroxypropyl)-N-hydroxyethylhexadecanamide).

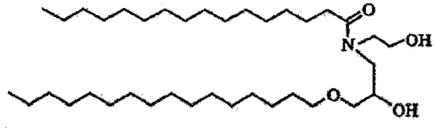

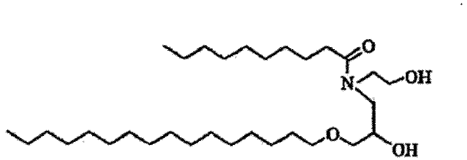

Examples of the alcohols having from 8 to 22 carbon atoms include myristyl alcohol, cetyl alcohol, stearyl alcohol, behenyl alcohol, and oleyl alcohol. Of these, those having a linear alkyl group are preferable, alcohols having from 16 to 18 carbon atoms are more preferable, one or more selected from the group consisting of cetyl alcohol and stearyl alcohol are further more preferable, and it is even more preferable to include cetyl alcohol and stearyl alcohol.

Examples of the glycerol fatty acid esters having from 8 to 22 carbon atoms include glycerol monocaprylate, glycerol monolaurate, glycerol monomyristate, glycerol monopalmitate, glycerol monostearate, glycerol monobehenate, glycerol monooleate, glycerol monoisostearate, and glycerol dicaprylate. Of these, one or more selected from the group consisting of glycerol monocaprylate, glycerol monooleate, glycerol monobehenate, glycerol monostearate, and glycerol monopalmitate are preferable, and glycerol monocaprylate, glycerol monooleate, and glycerol monostearate are more preferable.

Examples of the alkyl glyceryl ethers having from 8 to 22 carbon atoms include monodecyl glyceryl ether, monolauryl glyceryl ether, monomyristyl glyceryl ether, monocetyl glyceryl ether, monostearyl glyceryl ether, and monobehenyl glyceryl ether.

Examples of the sorbitan fatty acid esters having from 8 to 22 carbon atoms include sorbitan monolaurate, sorbitan monomyristate, sorbitan monopalmitate, sorbitan monostearate, sorbitan monooleate, sorbitan monobehenate, and sorbitan dioleate, and suitable examples include sorbitan monooleate, sorbitan dioleate, and sorbitan monostearate.

Examples of the polyoxyethylene sorbitan fatty acid esters having from 8 to 22 carbon atoms include polyoxyethylene sorbitan monolaurate, polyoxyethylene sorbitan monomyristate, polyoxyethylene sorbitan monopalmitate, polyoxyethylene sorbitan monostearate, and polyoxyethylene sorbitan monobehenate.

Of these, one or more selected from the group consisting of ceramides, sorbitan fatty acid esters having from 8 to 22 carbon atoms, and polyoxyethylene sorbitan fatty acid esters having from 8 to 22 carbon atoms are preferable as the component (b) from the viewpoint of stably dispersing the component (a) to impart a high ultraviolet protective effect, and of imparting a good impression from use. Furthermore, one or more selected from the group consisting of ceramides, sorbitan mono-fatty acid esters having from 14 to 22 carbon atoms, and polyoxyethylene sorbitan mono-fatty acid esters having from 14 to 22 carbon atoms are more preferable as the component (b).

A content of the component (b) in the water-in-oil emulsion cosmetic of the present invention is preferably 0.5 mass% or more, more preferably 0.8 mass% or more, and further more preferably 1 mass% or more from the viewpoint of stably dispersing the component (a) to impart a high ultraviolet protective effect, and of imparting a good impression from use. From the same viewpoint, it is preferably 7.5 mass% or less, more preferably 5 mass% or less, and further more preferably 3 mass% or less. Specifically, the content of the component (b) is preferably 0.5 mass% or more and 7.5 mass% or less, more preferably 0.8 mass% or more and 5 mass% or less, and further more preferably 1 mass% or more and 3 mass% or less.

The component(c) is a linear silicone oil.

A linear silicone oil is a silicone oil whose main chain is a linear dimethylpolysiloxane, and includes silicone oils containing alkyl groups, phenyl groups, and the like in the side chain. The linear silicone oil may not be limited as long as it is in a liquid state at normal temperature. Specific examples thereof include dimethylpolysiloxane, alkyl modified silicone oil, and phenyl modified silicone oil. Suitable examples include dimethylpolysiloxane having a kinematic viscosity of 1 mm²/s or more and 20 mm²/s or less at 25°C, and more suitable examples include dimethylpolysiloxane having a kinematic viscosity of 1 mm²/s or more and 10 mm²/s or less at 25°C.

A content of the component (c) in the water-in-oil emulsion cosmetic of the present invention is preferably 10 mass% or more, more preferably 15 mass% or more, and further more preferably 20 mass% or more from the viewpoint of stably dispersing the component (a) to impart a high ultraviolet protective effect, and of imparting a good impression from use such as the spreadability of the cosmetic. From the same viewpoint, it is preferably 50 mass% or less, more preferably 40 mass% or less, and further more preferably 35 mass% or less. Specifically, the content of the component (c) is preferably 10 mass% or more and 50 mass% or less, more preferably 15 mass% or more and 40 mass% or less, and further more preferably 20 mass% or more and 35 mass% or less.

The component (d) is a volatile hydrocarbon oil.

Examples of the volatile hydrocarbon oil used in the present invention include hydrocarbon oils having a flash point of 35°C or more and 100°C or less. Specifically, one or more selected from the group consisting of hydrogenated polyisobutene and isododecane can be used, and those usually commercially available as light liquid isoparaffin can be used.

By using (d) a volatile hydrocarbon oil as a volatile oil in addition to the component (b) and the component (c), the component (a) can be more stably dispersed in the oil component containing the component (b), and a water-in-oil emulsion cosmetic can be obtained, in which an excellent ultraviolet protective effect is stably imparted, whitishness is suppressed, and good spreadability and stability are imparted.

A content of the component (d) in the water-in-oil emulsion cosmetic of the present invention is preferably 1 mass% or more, more preferably 2 mass% or more, and further more preferably 3 mass% or more from the viewpoint of more stably dispersing the component (a) to impart a high ultraviolet protective effect, and of imparting a good impression from use such as the spreadability of the cosmetic. From the same viewpoint, it is preferably 10 mass% or less, more preferably 8 mass% or less, and further more preferably 7 mass% or less. Specifically, the content of the component (d) is preferably 1 mass% or more and 10 mass% or less, more preferably 2 mass% or more and 8 mass% or less, and further more preferably 3 mass% or more and 7 mass% or less.

Other than the above described components, the water-in-oil emulsion cosmetic of the present invention can contain an oil other than the above component (c) and component (d), a surfactant other than the above component (b), a thickener, a moisturizing agent, a powder other than the above component (a), alcohols, a chelating agent, a pH adjuster, a preservative, a dye, a fragrance, an ultraviolet absorber, water, and the like in combination.

In the water-in-oil emulsion cosmetic of the present invention, a high ultraviolet protective effect can be imparted even when a content of the ultraviolet absorber is lowered or no ultraviolet absorber is contained. The content of the ultraviolet absorber in the water-in-oil emulsion cosmetic of the present invention is preferably 0 mass% or more and 20 mass% or less, more preferably 0 mass% or more and 12 mass% or less, and can furthermore be 0 mass% or more and 5 mass% or less, 0 mass% or more and 2.5 mass% or less, 0 mass% or more and 1 mass% or less, and 0 mass% or more and 0.5 mass% or less.

Examples of oils other than the above include non-silicone oils in a liquid state at normal temperature, such as non-volatile hydrocarbon oils such as squalane, liquid isoparaffin, and liquid paraffin, and ester oils such as cetyl 2-ethylhexanoate, isononyl isononanoate, isotridecyl isononanoate, isopropyl myristate, isopropyl palmitate, 2-ethylhexyl palmitate, 2-ethylhexyl stearate, stearyl stearate, C12-15 alkyl benzoate, caprylic/capric triglyceride, glyceryl tri(2-ethylhexanoate), neopentyl glycol dicaprate, and neopentyl glycol diethylhexanoate.

The surfactant may not be limited as long as it is capable of forming a water-in-oil emulsion system, but nonionic surfactants are preferable. Furthermore, lipophilic nonionic surfactants having an HLB of 8 or less are preferable, and examples thereof include polyoxyethylene fatty acid esters, polyoxyethylene alkyl ethers, polyoxyethylene methylpolysiloxane copolymers, glycerol esters of fatty acids, glycerol alkyl ethers, sorbitan fatty acid esters, and polyether modified silicone.

A content of these surfactants in the water-in-oil emulsion cosmetic of the present invention is preferably 0 mass% or more and 5 mass% or less, more preferably 0.1 mass% or more and 4 mass% or less, and further more preferably 0.3 mass% or more and 3 mass% or less.

As the thickener, an oil-soluble thickener is preferable. The oil-soluble thickener is preferably a dextrin fatty acid ester, and examples thereof include esters of dextrin and fatty acids having from 10 to 24 carbon atoms, such as dextrin laurate, dextrin palmitate, dextrin myristate, dextrin stearate, dextrin isostearate, dextrin behenate, and dextrin cocoate. Of these, dextrin palmitate is preferable from the viewpoint of stability.

For example, commercial products such as Rheopearl KL2, Rheopearl TL, and UNIFILMA HVY (all of the above are manufactured by Chiba Flour Milling) can be used as the dextrin fatty acid ester.

The thickener can be used alone or in combination of two or more, and a content thereof in the water-in-oil emulsion cosmetic of the present invention is preferably 0.01 mass% or more and 2 mass% or less, and more preferably 0.05 mass% or more and 1 mass% or less.

The aqueous phase of the water-in-oil emulsion cosmetic of the present invention can contain polyhydric alcohols such as glycerin, 1,3-butylene glycol, propylene glycol, dipropylene glycol, and polyethylene glycol, plant extracts, and the like in addition to water.

A content of the component (e) in the water-in-oil emulsion cosmetic of the present invention is preferably 1 mass% or more, more preferably 2 mass% or more, and further more preferably 3 mass% or more from the viewpoint of spreadability and stability, and preferably 20 mass% or less, more preferably 15 mass% or less, and further more preferably 10 mass% or less from the same viewpoint. Specifically, it is preferably 1 mass% or more and 20 mass% or less, more preferably 2 mass% or more and 15 mass% or less, and further more preferably 3 mass% or more and 10 mass% or less.

A content mass ratio of the component (a) to the component (e), [(a)/(e)], is preferably 0.5 or more, more preferably 1 or more, further more preferably 2 or more, and preferably 10 or less, more preferably 6 or less, and further more preferably 5 or less from the viewpoint of stably dispersing the component (a) to stably impart a high ultraviolet protective effect, as well as suppressing whitishness, and having excellent spreadability and stability. As a specific range, 0.5 or more and 10 or less is preferable, 1 or more and 6 or less is more preferable, and 2 or more and 5 or less is further more preferable.

A content mass ratio of the component (b) to the component (e), [(b)/(e)], is preferably 0.03 or more, more preferably 0.06 or more, further more preferably 0.1 or more, and preferably 6 or less, more preferably 2 or less, and further more preferably 1 or less from the viewpoint of stably dispersing the component (a) to stably impart a high ultraviolet protective effect, as well as suppressing whitishness, and having excellent spreadability and stability. As a specific range, 0.03 or more and 6 or less is preferable, 0.06 or more and 2 or less is more preferable, and 0.1 or more and 1 or less is further more preferable.

The form of the cosmetic of the present invention is a water-in-oil emulsion cosmetic, which can be produced in accordance with conventional methods. For example, it can be produced by dispersing a powder component in an oil phase component, to which a previously mixed aqueous phase component is added, and then mixing and stirring the mixture. More preferably, from the viewpoint of stably dispersing the component (a) to impart a high ultraviolet protective effect, it is preferable to disperse the component (a) in the non-volatile oil including the component (b) which are being heated for dissolution, and then mix it with the other oils including the component (c) and the component (d), followed by cooling and water-in-oil emulsification by mixing with an aqueous phase component.

Therefore, the present invention provides a water-in-oil emulsion cosmetic obtained by liquefying an oil phase containing the component (a) and the component (b) by heating, mixing the oil phase with the component (c) and the component (d), cooling the mixture, and mixing the mixture with an aqueous phase component:
(a) titanium dioxide surface-treated with a fatty acid and/or an alkylalkoxysilane;
(b) an amphiphilic substance which is solid at normal temperature;
(c) a linear silicone oil; and
(d) a volatile hydrocarbon oil.

Examples of the formulations of the water-in-oil emulsion cosmetic of the present invention include emulsion, cream, and gel formulations.

In the water-in-oil emulsion cosmetic of the present invention, titanium dioxide surface-treated with a fatty acid and/or an alkylalkoxysilane is uniformly dispersed as an emulsion in the base, which stably provides an excellent ultraviolet protective effect, suppresses whitishness, and provides good spreadability and stability. Therefore, the water-in-oil emulsion cosmetic of the present invention is especially useful as a sunscreen cosmetic.

In relation to the above embodiments, the present invention further discloses the following compositions and methods.
<1> A water-in-oil emulsion cosmetic comprising the following components (a), (b), (c) and (d):
   (a) titanium dioxide surface-treated with a fatty acid and/or an alkylalkoxysilane;
   (b) an amphiphilic substance in a solid state at normal temperature;
   (c) a linear silicone oil; and
   (d) a volatile hydrocarbon oil.
<2> The water-in-oil emulsion cosmetic according to <1>, wherein a content of the component (a) is 3 mass% or more and 20 mass% or less, preferably 5 mass% or more and 20 mass% or less, and more preferably 5 mass% or more and 15 mass% or less.
<3> The water-in-oil emulsion cosmetic according to <1> or <2>, wherein an average particle size of titanium dioxide in the component (a) is 5 nm or more and 100 nm or less, preferably 5 nm or more and 50 nm or less, and more preferably 10 nm or more and 30 nm or less.
<4> The water-in-oil emulsion cosmetic according to any one of <1> to <3>, further comprising hydrophobically-treated zinc oxide.
<5> The water-in-oil emulsion cosmetic according to <4>, wherein a content of hydrophobically-treated zinc oxide is 5 mass% or more and 30 mass% or less, preferably 8 mass% or more and 25 mass% or less, and more preferably 10 mass% or more and 23 mass% or less.
<6> The water-in-oil emulsion cosmetic according to any one of <1> to <5>, wherein the component (b) is one or more selected from the group consisting of ceramides, alcohols having from 8 to 22 carbon atoms, glycerol fatty acid esters having from 8 to 22 carbon atoms, alkyl glyceryl ethers having from 8 to 22 carbon atoms, sorbitan fatty acid esters having from 8 to 22 carbon atoms, and polyoxyethylene sorbitan fatty acid esters having from 8 to 22 carbon atoms.
<7> The water-in-oil emulsion cosmetic according to any one of <1> to <6>, wherein a content of the component (b) is 0.5 mass% or more and 7.5 mass% or less, preferably 0.8 mass% or more and 5 mass% or less, and more preferably 1 mass% or more and 3 mass% or less.
<8> The water-in-oil emulsion cosmetic according to any one of <1> to <7>, wherein a component (c) is a linear silicone oil in a liquid state at normal temperature.
<9> The water-in-oil emulsion cosmetic according to any one of <1> to <8>, wherein a content of the component (c) is 10 mass% or more and 50 mass% or less, preferably 15 mass% or more and 40 mass% or less, and more preferably 20 mass% or more and 35 mass% or less.
<10> The water-in-oil emulsion cosmetic according to any one of <1> to <9>, wherein the component (d) is one or more selected from the group consisting of hydrogenated polyisobutene and isododecane.
<11> The water-in-oil emulsion cosmetic according to any one of <1> to <10>, wherein a content of the component (d) is 1 mass% or more and 10 mass% or less, preferably 2 mass% or more and 8 mass% or less, and more preferably 3 mass% or more and 7 mass% or less.
<12> The water-in-oil emulsion cosmetic according to any one of <1> to <11>, further comprising an ultraviolet absorber.
<13> The water-in-oil emulsion cosmetic according to <12>, wherein a content of the ultraviolet absorber is 0 mass% or more and 20 mass% or less, preferably 0 mass% or more and 12 mass% or less, more preferably 0 mass% or more and 5 mass% or less, further more preferably 0 mass% or more and 2.5 mass% or less, further more preferably 0 mass% or more and 1 mass% or less, and further more preferably 0 mass% or more and 0.5 mass% or less.
<14> The water-in-oil emulsion cosmetic according to any one of <1> to <13>, further comprising one or more selected from the group consisting of non-silicone oils in a liquid state at normal temperature, non-volatile hydrocarbon oils, and ester oils.
<15> The water-in-oil emulsion cosmetic according to any one of <1> to <14>, further comprising a nonionic surfactant.
<16> The water-in-oil emulsion cosmetic according to any one of <1> to <15>, further comprising a thickener.
<17> The water-in-oil emulsion cosmetic according to any one of <1> to <16>, further comprising water as a component (e).
<18> The water-in-oil emulsion cosmetic according to <17>, wherein a content of the component (e) water is 1 mass% or more and 20 mass% or less, preferably 2 mass% or more and 15 mass% or less, and more preferably 3 mass% or more and 10 mass% or less.
<19> The water-in-oil emulsion cosmetic according to <17> or <18>, wherein a content mass ratio of the component (a) to the component (e), [(a)/(e)], is 0.5 or more and 10 or less, preferably 1 or more and 6 or less, and more preferably 2 or more and 5 or less.
<20> The water-in-oil emulsion cosmetic according to any one of <17> to <19>, wherein a content mass ratio of the component (b) to the component (e), [(b)/(e)], is 0.03 or more and 6 or less, preferably 0.06 or more and 2 or less, and more preferably 0.1 or more and 1 or less.
<21> The water-in-oil emulsion cosmetic according to any one of <1> to <20>, which is a water-in-oil emulsion sunscreen cosmetic.
<22> A water-in-oil emulsion cosmetic, produced by
   liquefying an oil phase comprising a component (a) and a component (b) by heating,
   mixing the oil phase with a component (c) and a component (d),
   cooling the mixture, and
   mixing the mixture with an aqueous phase component:
      (a) titanium dioxide surface-treated with a fatty acid and/or an alkylalkoxysilane;
      (b) an amphiphilic substance in a solid state at normal temperature;
      (c) a linear silicone oil; and
      (d) a volatile hydrocarbon oil.
<23> A method for producing a water-in-oil emulsion cosmetic, comprising
   liquiefying an oil phase containing a component (a) and a component (b) by heating,
   mixing the oil phase with a component (c) and a component (d),
   cooling the mixture,
   mixing the mixture with an aqueous phase component:
      (a) titanium dioxide surface-treated with a fatty acid and/or an alkylalkoxysilane;
      (b) an amphiphilic substance which is solid at normal temperature;
      (c) a linear silicone oil; and
      (d) a volatile hydrocarbon oil.

### Examples

Next, the present invention will be described in more detail using Examples. In the following Examples, a content of each component is indicated as a mass% of the total composition.

Emulsions, which were water-in-oil emulsion cosmetics, were produced using the prescriptions shown in Tables 1 and 2, by the preferable method described above, and the following evaluations were performed.

### (1) Ultraviolet Protection Ability

28.5 g of the emulsions obtained in the Examples and Comparative Examples were uniformly applied to PAMM plates (5 cm × 5 cm), allowed to dry for 15 minutes, and then the spectra at 9 points on the PAMM plate were measured by an SPF analyzer (UV2000s, manufactured by Labsphere). The mean value was calculated and assessed in accordance with the following evaluation criteria.
a: in vitro SPF of 100 or more
b: in vitro SPF of 60 or more and less than 100
c: in vitro SPF of 40 or more and less than 60
d: in vitro SPF of less than 40

### (2) Spreadability of Cosmetic

The viscosity of the emulsions obtained in the Examples and Comparative Examples was measured at room temperature using a B-type viscometer (No. 3-6 rpm) and assessed in accordance with the following evaluation criteria.
a: Viscosity of less than 5,000 mPa·s
b: Viscosity of 5,000 mPa·s or more and less than 6,000 mPa·s
c: Viscosity of 6,000 mPa·s or more and less than 8,000 mPa·s
d: Viscosity of 8,000 mPa·s or more

### (3) Whitishness

0.2 mL of the emulsions obtained in the Examples and Comparative Examples was dropped on a black leather (12 cm × 7 cm) and uniformly applied with a 25 µm bar coater. ΔL was measured with a colorimeter and assessed in accordance with the following evaluation criteria.
a: ΔL of less than 3
b: ΔL of 3 or more and less than 5
c: ΔL of 5 or more and less than 6
d: ΔL of 6 or more

**[Table 1]**

| Component (Mass%) | | **Example** | | | | | | | | | | |
|---|---|---|---|---|---|---|---|---|---|---|---|---|
| | | 1 | 2 | 3 | 4 | 5 | 6 | 7 | 8 | 9 | 10 | 11 |
| (b) | Pseudo-ceramide *1 | 1 | 1 | 1 | 1 | 1 | 1 | 1 | 1 | 1.5 | | |
| | Polyoxyethylene Sorbitan Monostearate (20 E.O.) *2 | | | | | | | | | | 2 | |
| | Sorbitan Monostearate *3 | | | | | | | | | | | 2 |
| | PEG-3 Dimethicone *4 | 3 | 3 | 3 | 3 | 3 | 3 | 3 | 3 | | | |
| | Dextrin Palmitate *5 | | | | | | | 0.2 | | | | |
| | Squalane *6 | 5 | 5 | 5 | 5 | 5 | 5 | 5 | 5 | 5 | 5 | 5 |
| | Liquid Isoparaffin *7 | 10 | 10 | 10 | 10 | 10 | 25 | 9.8 | 10 | 12.5 | 12 | 12 |
| (d) | Hydrogenated Polyisobutene *8 | 5 | 5 | 5 | | 5 | 5 | 5 | 5 | 5 | 5 | 5 |
| | Isododecane *9 | | | | 5 | | | | | | | |
| | Neopentyl Glycol Dicaprate *10 | 3 | 3 | 3 | 3 | 3 | 3 | 3 | 3 | 3 | 3 | 3 |
| (c) | Dimethicone *11 | 30 | 30 | 30 | 30 | 30 | 30 | 30 | 30 | 30 | 30 | 30 |
| (a) | Stearic Acid-Treated Titanium dioxide *12 | 10 | 10 | 10 | 10 | | | 10 | | 10 | 10 | 10 |
| | OTS-Treated Titanium dioxide *13 | | | | | 10 | 15 | | | | | |
| | Isostearic Acid-Treated Titanium dioxide *14 | | | | | | | | 10 | | | |
| | Dimethicone-Treated Titanium dioxide *15 | | | | | | | | | | | |
| | Dimethicone-Treated Zinc Oxide *16 | 20 | | | | | | | | | | |
| | Dimethicone/Methicone Mix-Treated Zinc Oxide *17 | | 20 | | 20 | 20 | | 20 | 20 | 20 | 20 | 20 |
| | OTS-Treated Zinc Oxide *18 | | | 20 | | | | | | | | |
| | Glycerin *19 | 1 | 1 | 1 | 1 | 1 | 1 | 1 | 1 | 1 | 1 | 1 |
| | 1,3-Butylene Glycol *20 | 8 | 8 | 8 | 8 | 8 | 8 | 8 | 8 | 8 | 8 | 8 |
| | Purified Water | 4 | 4 | 4 | 4 | 4 | 4 | 4 | 4 | 4 | 4 | 4 |
| | Total | 100 | 100 | 100 | 100 | 100 | 100 | 100 | 100 | 100 | 100 | 100 |
| Results | Ultraviolet Protection Ability | b | b | a | b | a | c | b | b | a | b | b |
| | Spreadability of Cosmetic | a | a | a | a | a | a | a | a | b | a | a |
| | Whitishness | b | b | b | b | b | a | b | b | b | b | b |

**[Table 2]**

| Component (Mass%) | | **Comparative Example** | | | | |
|---|---|---|---|---|---|---|
| | | **1** | **2** | **3** | **4** | **5** |
| (b) | Pseudo-ceramide *1 | | 1 | 1 | | 1 |
| | Polyoxyethylene Sorbitan Monostearate (20 E.O.) *2 | | | | | |
| | Sorbitan Monostearate *3 | | | | | |
| | PEG-3 Dimethicone *4 | 4 | 3 | 3 | 4 | 3 |
| | Dextrin Palmitate *5 | | | | | |
| | Squalane *6 | 5 | 5 | 5 | 5 | 5 |
| | Liquid Isoparaffin *7 | 10 | 15 | 40 | 10 | 10 |
| (d) | Hydrogenated Polyisobutene *8 | 5 | | 5 | | 5 |
| | Isododecane *9 | | | | 5 | |
| | Neopentyl Glycol Dicaprate *10 | 3 | 3 | 3 | 3 | 3 |
| (c) | Dimethicone *11 | 30 | 30 | | 30 | 30 |
| (a) | Stearic Acid-Treated Titanium dioxide *12 | 10 | 10 | 10 | 10 | |
| | OTS-Treated Titanium dioxide *13 | | | | | |
| | Isostearic Acid-Treated Titanium dioxide *14 | | | | | |
| | Dimethicone-Treated Titanium dioxide *15 | | | | | 10 |
| | Dimethicone-Treated Zinc Oxide *16 | | | | | |
| | Dimethicone/Methicone Mix-Treated Zinc Oxide *17 | 20 | 20 | 20 | 20 | 20 |
| | OTS-Treated Zinc Oxide *18 | | | | | |
| | Glycerin *19 | 1 | 1 | 1 | 1 | 1 |
| | 1,3-Butylene Glycol *20 | 8 | 8 | 8 | 8 | 8 |
| | Purified Water | 4 | 4 | 4 | 4 | 4 |
| | Total | 100 | 100 | 100 | 100 | 100 |
| Results | Ultraviolet Protection Ability | c | b | a | c | d |
| | Spreadability of Cosmetic | a | a | d | a | a |
| | Whitishness | d | d | a | c | b |

*1: N-(hexadecyloxyhydroxypropyl)-N-hydroxyethylhexadecanamide (Sphingolipid E, manufactured by Kao)
*2: RHEODOL TW-S120V (manufactured by Kao)
*3: RHEODOL SP-S10V (manufactured by Kao)
*4: Silicone KF-6015 (manufactured by Shin-Etsu Chemical)
*5: Rheopearl KL2 (manufactured by Chiba Flour Milling)
*6: NIKKOL Squalane (manufactured by NIPPON SURFACTANT INDUSTRIES)
*7: PARLEAM EX (manufactured by NOF)
*8: PARLEAM 4 (manufactured by NOF)
*9: Creasil ID CG (manufactured by Cosmetics Innovations and Technologies Sarl)
*10: ESTEMOL N-01 (manufactured by The Nisshin Oillio Group)
*11: Silicone KF-96L-2CS (-G) (manufactured by Shin-Etsu Chemical)
*12: MT-100TV (manufactured by TAYCA)
*13: STR-100W-OTS (manufactured by SAKAI CHEMICAL INDUSTRY)
*14: MT-150EX (manufactured by TAYCA)
*15: MTY-100SAS (manufactured by TAYCA)
*16: MZX-300M (manufactured by TAYCA)
*17: MZX-304R3M (manufactured by TAYCA)
*18: MZX-304OTS (manufactured by TAYCA)
*19: 86% Glycerin V (manufactured by Kao)
*20: 1,3-Butylene Glycol-P (manufactured by KH Neochem)

According to Tables 1 and 2, the water-in-oil emulsion cosmetic containing the components (a), (b), (c), and (d) of the present invention exhibited very high ultraviolet protection ability, the skin on which it was applied did not turn whitish, and the cosmetic spreadability was good, as well as impression from use.

Uniform dispersion of the component (a) of the present invention in the base was confirmed by microscopic observation.

2.5 mass% of a pseudo-ceramide (Sphingolipid E, manufactured by Kao) was dissolved by heating in 14 mass% of liquid isoparaffin (PARLEAM EX, manufactured by NOF) and 5 mass% of neopentyl glycol dicaprate (ESTEMOL N-01, manufactured by The Nisshin Oillio Group), to which 12 mass% of stearic acid-treated titanium dioxide (MT-100Z, manufactured by TAYCA) was added. Then, the mixture was dispersed for 15 minutes with disper (2,500 rpm), to which 66.5 mass% of dimethicone (Silicone KF-96L-2CS (-G), manufactured by Shin-Etsu Chemical) was added, and stirred with a propeller for 10 minutes.

The obtained dispersion was observed under a microscope. The results are shown in Figure 1.

Figure 2 shows the microscopic observation results of the dispersion obtained by using caprylic triglyceride (COCONAD RK, manufactured by Kao), which is an amphiphilic substance in a liquid state at normal temperature, instead of a pseudo-ceramide.

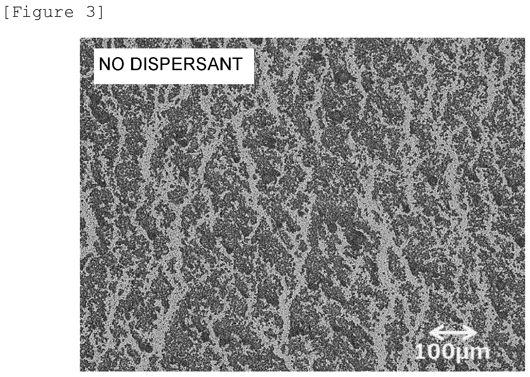

Figure 3 shows the microscopic observation results of the dispersion produced in the same manner except that no pseudo-ceramide (component (b)) was contained in the above dispersion.

The results of Figures 1 to 3 revealed that the dispersion effect of the component (a) is enhanced if an amphiphilic substance (component (b)) which is solid at normal temperature is contained.

## Claims

1. A water-in-oil emulsion cosmetic comprising the following components (a), (b), (c) and (d):
(a) titanium dioxide surface-treated with a fatty acid and/or an alkylalkoxysilane;
(b) an amphiphilic substance which is solid at normal temperature;
(c) a linear silicone oil; and
(d) a volatile hydrocarbon oil.

2. The water-in-oil emulsion cosmetic according to claim 1, wherein the component (b) is one or more selected from the group consisting of ceramides, alcohols having from 8 to 22 carbon atoms, glycerol fatty acid esters having from 8 to 22 carbon atoms, alkyl glyceryl ethers having from 8 to 22 carbon atoms, sorbitan fatty acid esters having from 8 to 22 carbon atoms, and polyoxyethylene sorbitan fatty acid esters having from 8 to 22 carbon atoms.

3. The water-in-oil emulsion cosmetic according to claim 1 or 2, wherein the component (c) is a linear silicone oil in a liquid state at normal temperature.

4. The water-in-oil emulsion cosmetic according to any one of claims 1 to 3, wherein the component (d) is one or more selected from the group consisting of hydrogenated polyisobutene and isododecane.

5. The water-in-oil emulsion cosmetic according to any one of claims 1 to 4, wherein a content of the component (d) is 1 mass% or more and 10 mass% or less.

6. The water-in-oil emulsion cosmetic according to any one of claims 1 to 5, which is a water-in-oil emulsion sunscreen cosmetic.

7. A water-in-oil emulsion cosmetic, obtained by liquefying an oil phase comprising a component (a) and a component (b) by heating, mixing the oil phase with a component (c) and a component (d), cooling the mixture, and mixing the mixture with an aqueous phase component:
(a) titanium dioxide surface-treated with a fatty acid and/or an alkylalkoxysilane;
(b) an amphiphilic substance which is solid at normal temperature;
(c) a linear silicone oil; and
(d) a volatile hydrocarbon oil.
